# EUROPEAN PATENT APPLICATION

(11) **EP 4 732 753 A1**
(43) Date of publication of application: **29.04.2026**
(21) Application number: 24208889.6
(22) Date of filing: 25.10.2024
(51) Int. Cl.: A61B 3/10

(54) **POLYGON SCANNING MIRROR APERTURE IN AN OPHTHALMIC IMAGING INSTRUMENT**

(71) Applicant: Optos plc, Dunfermline, Scotland KY11 8GR (GB)
(72) Inventor: SWAN, Derek, Dunfermline, Scotland, KY11 8GR (GB); MUYO, Gonzalo, Dunfermline, Scotland, KY11 8GR (GB)
(74) Representative: Hoffmann Eitle

(57) **Abstract**

An ophthalmic imaging instrument (102, 120) comprising a light source (200) which emits a beam of light (201), a polygon scanning mirror (202) comprising a plurality of reflecting facets (2021, 2022) each extending in a respective first direction (d1) in a plane of rotation of said mirror (202) and in a common orthogonal second direction (d2), a driver (203) arranged to rotate said mirror (202) such that each facet (2021, 2022) reflects the beam of light (201), an optical element (204) which guides the reflected beam towards an eye (E) and guides light (LR) returning from the eye (E) towards the mirror (202), a detector (205) which detects the returning light (LR) reflected from the mirror (202), and an aperture (206) which constrains the returning light (LR) reaching the detector (205) to be that reflected from a region (2021A) of each facet (2021, 2022) which extends at least one of a same first distance on each facet in its respective first direction (d1) and a same second distance on each facet in the common second direction (d2).

## Description

### Technical field

The present invention relates to a polygon scanning mirror in an ophthalmic imaging instrument. More particularly but not exclusively, the present invention relates to an aperture arranged to constrain light returning from an eye so as to reduce a variation among the facets of the polygon scanning mirror in the amount of the returning light L_{R} reflected from each facet which reaches the detector to form an image of the eye.

### Background

Ophthalmic imaging instruments are widely used to image a patient's eye in order to assess the health of the eye. Such instruments typically comprise a light source and a controller that is arranged to control the light source to generate a beam of light of a target optical power. The beam of light is deflected by one or more scan relay elements so as to cover an imaging area by means of a typically parallel arrangement of a plurality of scanning lines. The scan relay element(s) usually comprise a polygon scanning mirror and one or more scanning galvanometer scanning mirrors, herein referred to as "galvos". The former polygon scanning mirror comprises a plurality of reflecting facets that are arranged along an outer circumference of a rotating body. A driver is operable to rotate the body of the polygon scanning mirror, such that each facet successively reflects an incident beam of light at a varying angle. Thus, during the passing of one individual facet, the beam of light is deflected through the varying angle. After completion of such a deflection cycle, the next facet substantially repeats this process and so do all other facets.

This repeated deflection of the beam of light by the polygon scanning mirror forms the basis for at least one scanning direction by means of providing a plurality of parallel scanning lines. A further scanning element, such as an already mentioned galvo, can then further deflect the beam in another direction so as to displace the scanning lines from each other on the target and to ultimately cover a two-dimensional scanning area. At least in some imaging modalities, the light from the scanning beam is scattered back from the target tissue which includes in the case of ophthalmic imaging primarily parts of the human eye such as, for example, the fundus or retina. In general, the light thus travels back along the incident light path so as to be ultimately detected by a detector in the form of a light sensor, converted into an intensity signal, and processed to form the image. Especially the latter signal processing is implemented by means of computing resources that compile individual images from corresponding sets of line scans.

The light returning from eye which reflects from each facet in turn is thus collected by intermediary optics, detected by the detector and used to generate the image. However, each facet often has slight variations in size and shape as a result of the manufacturing process used to produce the polygon scanning mirror. These variations may result in differing amounts of light being reflected from each of the facets and so differing amounts of light being collected and detected by the detector along each scan line, thereby resulting in the brightness of each scan line varying depending on which facet is used due to this differing amount of collected light. This can cause visible 'banding' in the generated images that is detrimental to the quality of the image.

Therefore, there is a need to control the amount of light detected by the detector along each scan line from facet to facet to reduce, or preferably eliminate, banding in the images, whilst keeping system complexity at an acceptable level.

### Summary

According to one embodiment of the present invention, there is provided an ophthalmic imaging instrument. The ophthalmic imaging instrument comprises a light source, a polygon scanning mirror, a driver, an optical element, a detector, an aperture and an access to computing resources. The light source is arranged to emit a beam of light. The polygon scanning mirror comprises a plurality of reflecting facets each extending in a respective first direction in a plane of rotation of the polygon scanning mirror and in a common second direction orthogonal to the plane of rotation of the polygon scanning mirror. The driver is arranged to rotate during operation said polygon scanning mirror in the plane of rotation, such that each facet reflects the beam of light. The optical element is arranged to guide the beam reflected from the polygon scanning mirror towards an eye of a subject and to guide light returning from the eye towards the polygon scanning mirror. The detector is arranged to detect the returning light reflected from the polygon scanning mirror to generate a detection signal. The aperture is arranged to constrain the returning light reaching the detector to be that reflected from a region of each facet which extends at least one of a same first distance on each facet in its respective first direction and/or a same second distance on each facet in the common second direction. The access is to computing resources which are arranged to generate an image of the eye using the detection signal generated from the returning light reflected by at least one facet.

According to a further embodiment of the present invention there is provided a method for operating the ophthalmic imaging instrument described above. The method comprises a step of rotating, by means of the driver, said polygon scanning mirror such that each facet reflects the beam of light, a step of guiding, by means of the optical element, the beam reflected from the polygon scanning mirror towards the eye of the subject, a step of guiding, by means of the optical element, light returning from the eye towards the polygon scanning mirror, a step of generating, by means of the detector, the detection signal by detecting the returning light constrained by the aperture, and a step of generating, by means of the access to the computing resources, the image of the eye using the detection signal generated from the returning light reflected by at least one facet.

### Brief Description of the Drawings

Embodiments of the present invention, which are presented for better understanding of the inventive concepts, but which are not to be seen as limiting the invention, will now be described with reference to the figures in which:
- Figure 1: shows a schematic view of an ophthalmic imaging instrument according to a general device embodiment of the present invention;
- Figure 2A: shows a schematic view of an ophthalmic imaging instrument according to a device embodiment of the present invention;
- Figure 2B: shows a schematic view of an alternative arrangement of the optical element according to the device embodiment of the present invention;
- Figures 3A & 3B: show schematic views of the rotating polygon mirror and an aperture according to the device embodiment of the present invention;
- Figure 4: shows a schematic view of an ophthalmic imaging instrument especially in the context of examples of further components and optical elements according to an embodiment of the present invention; and
- Figure 5: shows a flowchart of a general method embodiment of the present invention.

It should be understood that some of the drawings may not necessarily be shown to scale, unless otherwise indicated. In certain instances, details that are not necessary for an understanding of the disclosure or that render other details difficult to perceive may have been omitted. It should be understood, of course, that the disclosure is not necessarily limited to the particular examples or embodiments illustrated or depicted herein.

### Detailed Description

The present inventors have recognised that the aforementioned visible 'banding' in the images can be reduced, or even eliminated, by use of an aperture to constrain the amount of light reaching the detector to be that reflected from a region of each facet of a controlled size. In this way, variations in the size and shape of the facets can be reduced, or even removed, by virtue of the aperture which thereby ensures that a similar, or the same, amount of light reaches the detector when reflected from each facet in turn.

Example embodiments herein will now be described with reference to the accompanying drawings.

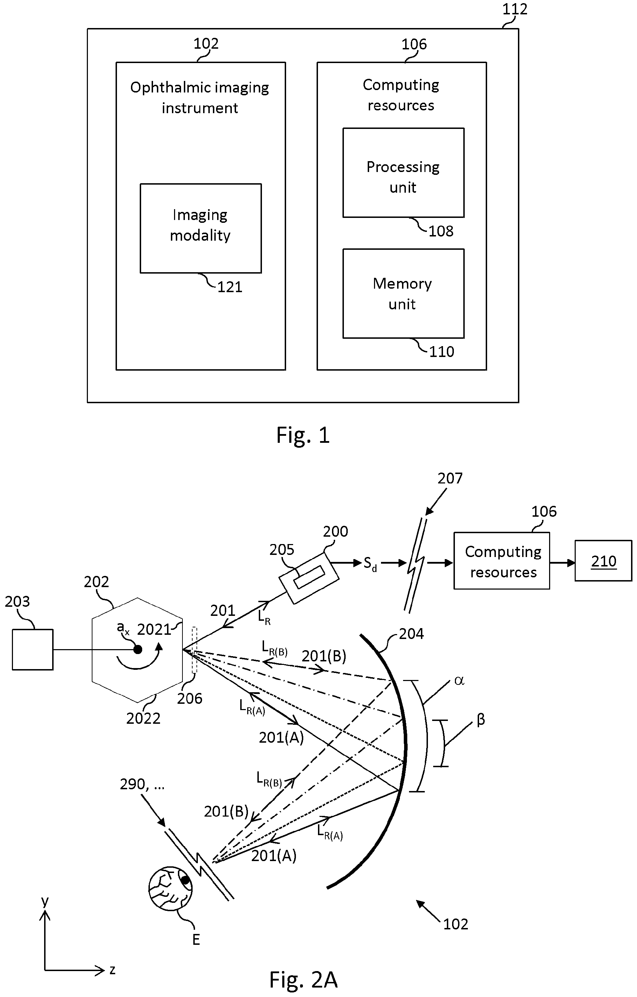

Figure 1 shows a schematic view of an ophthalmic imaging instrument 102 according to a general device embodiment of the present invention. Specifically, there is shown an ophthalmic imaging system 112 which includes the ophthalmic imaging instrument 102 providing at least one imaging modality 121 such as, for example, autofluorescence (AF), colour red-green, colour red-green-blue and fluorescein angiography. The ophthalmic imaging instrument 102 may be, or comprise, a scanning laser ophthalmoscope (SLO) so as to provide one or more of the at least one imaging modalities 121 and may comprise an optical coherence tomography (OCT) device so as to provide an OCT imaging modality. In some embodiments, the OCT device may share one or more optical components with the SLO such as, for example, the scanning system therein. The ophthalmic imaging system 112 may comprise or have access to (e.g. via a wired or wireless connection) computing resources 106 which, in turn, comprise a processing unit 108 and a memory unit 110. The components of the ophthalmic imaging system 112 including the ophthalmic imaging instrument 102 and the computing resources 106 may be housed inside a common housing so that the system 112 thereby forms the ophthalmic imaging instrument 102, such as an ophthalmoscope. In this way, the ophthalmic imaging instrument 102 has access to, or itself comprises, the computing resources 106. In some embodiments, the computing resources 106 may be located external to the instrument 102 within respective different housings. In some embodiments, any of the components may be located in a different housing from the rest of the components.

The computing resources 106 are operable to generate an image of the eye using a detection signal from a detector in the ophthalmic imaging instrument 102, as later described, and may be operable to control and provide the imaging modality 121. The computing resources have at least one processing unit 108 such as a central processing unit (CPU) and/or graphics processing unit (GPU), and a memory unit 110 that stores instructions that, when executed by the at least one processing unit 108, causes the processing unit 108 to perform one or more methods and functions described herein. In embodiments of local computing resources or local physical components of a computing device, the resources may include a processor, such as CPU and/or GPU, a system memory, and a system bus that couples the system memory to the CPU/GPU. The system memory may include a random access memory ("RAM") and a read-only memory ("ROM"). A basic input/output ("I/O") system containing the basic routines that help to transfer information between elements within the computing device, such as during startup, is stored in the ROM. The computing resources may further include a mass storage device, which is able to store software instructions and data. The mass storage device may be connected to the CPU/GPU through a mass storage controller connected to the system bus. The mass storage device and its associated computer-readable data storage media may provide non-volatile, non-transitory storage for the computing resources 106. Although the description of computer-readable data storage media contained herein refers to a mass storage device, such as a hard disk or CD-ROM drive, it should be appreciated by those skilled in the art that computer-readable data storage media can be any available non-transitory, physical device or article of manufacture from which the device can read data and/or instructions. The mass storage device is an example of a computer-readable storage device.

Computer-readable data storage media include volatile and non-volatile, removable and non-removable media implemented in any method or technology for storage of information such as computer-readable software instructions, data structures, program modules or other data. Example types of computer-readable data storage media include, but are not limited to, RAM, ROM, EPROM, EEPROM, flash memory or other solid-state memory technology, CD-ROMs, digital versatile discs ("DVDs"), other optical storage media, magnetic cassettes, magnetic tape, magnetic disk storage or other magnetic storage devices, or any other medium which can be used to store the desired information and which can be accessed by the computing device.

The computing resources 106 may operate in a networked environment using logical connections to remote network devices through a network, such as a local network, the Internet, or another type of network. The computing resources 106 may connect to the network through a network interface unit connected to the system bus. The network interface unit may also connect to other types of networks and remote computing systems. The computing resources 106 may include an input/output controller for receiving and processing input from a number of other devices, including a touch user interface display screen, or another type of input device. Similarly, the input/output controller may provide output to a touch user interface display screen, a printer, or other type of output device. As mentioned above, the mass storage device and the RAM can store software instructions and data. The software instructions may include an operating system suitable for controlling the operation of the computing resources 106. The mass storage device and/or the RAM may also store software instructions, that when executed by the CPU/GPU, cause the computing resources 106 to provide the functionality discussed in this document, including the methods described herein and shown in the figures.

In some embodiments which implement imaging modalities 121 with the mentioned SLO, the SLO may be a confocal laser scanning microscope for diagnostic imaging of the retina of the eye. The imaging may be two-dimensional (2D) imaging and may use a laser light beam to scan across the retina in a raster pattern to illuminate successive elements of the retina, point-by-point. The light reflected from each retinal point may be captured by a photomultiplier. The output of the photomultiplier may be recorded and displayed in a digital format. In this manner, the SLO may be able to produce high-contrast, detailed images of the retina. In some embodiments, images are captured sequentially by one imaging modality using the SLO and by at least a further imaging modality using at least one of the SLO and the OCT device.

Figure 2A shows a schematic view of the ophthalmic imaging instrument 102 according to a device embodiment of the present invention. As shown, the ophthalmic imaging instrument 102 comprises a light source 200 arranged to emit a beam of light 201 as a scanning beam. A polygon scanning mirror 202 acts a first scanning element (or, in other terms, a first scan relay) and is arranged to rotate in a plane of rotation (i.e. parallel to the y-z plane) about an axis of rotation a_{X}. The polygon scanning mirror 202 comprises a plurality of reflecting facets 2021, 2022, ... (e.g. of the same shape) each extending in a respective first direction in a plane of rotation of the polygon scanning mirror 202 (i.e. a direction defining the edges of the polygon shape of the polygon scanning mirror in the y-z plane) and in a common second direction (i.e. the x-direction, common to all facets of the plurality of facets) orthogonal to the plane of rotation of the polygon scanning mirror 202. In some embodiments, the beam 201 may have a cross-sectional area which is a fraction of the area of the facets (e.g. less than 0.1% or 1% of the area of each of the facets). The polygon scanning mirror 202 is arranged to reflect the beam 201 towards an optical element 204 and to reflect returning light L_{R} from the eye E towards the detector 205, as later described. As shown, the beam 201 may, upon incidence with the polygon scanning mirror 202, be travelling with a component in the direction opposite to the positive y-direction. In the present embodiment, the polygon scanning mirror is a regular convex polygon with 6 rectangular facets, each having a length in the respective first direction of that facet which is defined by the lengths of two edges of the polygon scanning mirror 202 parallel to the plane of rotation of the polygon scanning mirror 202 and a width in the common second direction which is defined by the lengths of two edges of the polygon scanning mirror 202 orthogonal to this plane. However, other numbers of facets (e.g. 16 facets or at least four or five facets), shapes of facets or geometries of polygon may instead be used.

A driver 203 is arranged to rotate during operation said polygon scanning mirror 202 (in the plane of rotation of the polygon scanning mirror 202) such that each facet 2021, 2022, ... reflects the beam of light 201 at a varying angle α (when the facet is rotated over a predetermined range of angular positions). The driver 203 may, for example, be an electric motor connected to the polygon scanning mirror 202 and controlled by a controller of the ophthalmic imaging instrument 102 (which may be the computing resources 106). As illustrated, the path of the scanning beam 201 is shown in a one-dimensional (1D) scan produced during example anti-clockwise rotation (shown by the curved arrow) of the polygon scanning mirror 202. Path "A" is an example of the scanning beam 201 reflected from a respective facet 2021 of the polygon scanning mirror 202 at a starting point during rotation, i.e. when the facet 2021 is moved into the path of the beam 201 from the light source 200. Path "B" is an example of the scanning beam 201 reflected from the respective facet 2021 of the polygon scanning mirror 202 at a later point in time further down the rotation when the facet 2021 is moved out of the path of the beam 201 from the light source 200.

In this way, the polygon scanning mirror 202 reflects the beam 201 at (or through) a varying angle α from the active facet (i.e. the facet presently reflecting the beam 201 from the light source 200 to the optical element 204) as the beam 201 moves from one edge of the facet defining the facets length to the other. As the facet 2021 moves out of reach of the beam 201, the adjacent facet 2022 (i.e. the neighbouring/bordering facet to the facet 2021 in the opposite rotational direction to that of the polygon scanning mirror 202) repeats substantially the described reflection process such that each facet successively reflects the beam 201 at the varying angle α in turn. This varying angle α varies as governed by the length and rotation of the respective facet and the respective orientation of the facet relative to the incident beam 201 determines the reflection angle of the beam 201. The varying angle α includes a varying angle β as a part of the varying angle α. This varying angle β may, as in the present embodiment, be different to the varying angle α and, more specifically, be a subset of it (e.g. greater than 50, 70 or 90% and less than 95 or 100 % of the varying angle α, centred within the varying angle α).

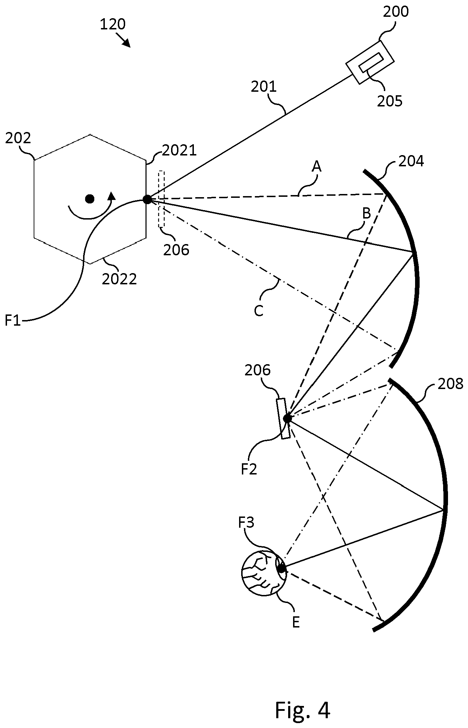

The ophthalmic imaging instrument 102 further comprises the optical element 204 arranged to guide the beam of light 201 that is reflected from the polygon scanning mirror 202 (i.e. the beam 201 reflected at the varying angle α) toward an eye E of a subject. As shown in Figure 2A, the optical element 204 is arranged such that the beam 201 is reflected toward the eye E of the subject as the beam 201 is reflected by the polygon scanning mirror 202 through the whole varying angle α. Further components and optical elements 290, ... may be arranged between the optical element 204 and the eye E of the subject, as described later with reference to Figure 4. The optical element 204 may be, or comprise, a curved mirror (e.g. an ellipsoidal mirror), as shown, having the active facet at one of its focal points but it may also take any other suitable forms. Embodiments considering a curved mirror are explained in greater detail elsewhere in the present disclosure.

The optical element 204 is further arranged to guide light L_{R} returning from the eye E towards the polygon scanning mirror 202. The returning light L_{R} may be light scattered from the eye E in, for example, the colour red-green or the colour red-green-blue imaging modality or may be, or comprise, light emitted from the eye E in, for example, the AF imaging modality. This return light L_{R} follows the same optical path as the beam 201 and so may be reflected by the optical element 204 towards the polygon scanning mirror 202 along optical paths corresponding to the reflection of the beam 201 at the varying angle α. The returning light L_{R} is thus guided by the optical element 204 to the active facet and this active facet reflects the returning light L_{R} towards a detector 205 along the optical path of the beam 201 so as to form an image 210 of the eye E, as later described. As the current active facet moves out of reach of the beam 201, the adjacent facet becomes the active facet and repeats substantially this described reflection process of the returning light L_{R} such that each facet reflects the returning light L_{R} towards the detector 205 along the optical path of the beam 201, in turn. Thus, successive scan lines in the image 210 may be formed by using, for example, an orthogonal scanning element, as later described. In some embodiments, the returning light L_{R} from the eye E may be a light beam of a diameter which is greater than that of the beam 201 and which may be (substantially) equal to that of the pupil of the eye E. The light beam may be of a size which (substantially) fills the entire area of each of the facets. However, it would be appreciated by those skilled in the art that this depends on the further components and optional elements 290, ..., the characteristics of the beam 201, the part of the eye E upon which the beam 201 is incident and the imaging modality 121, for example.

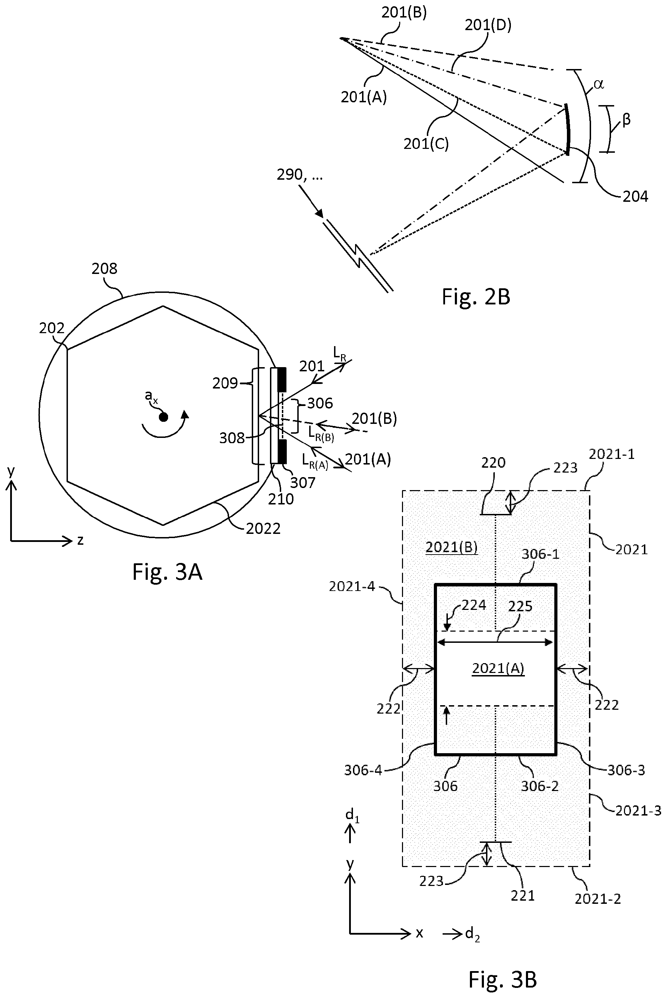

Figure 2B shows a schematic view of an alternative arrangement of the optical element 204 which may be used. In this alternative arrangement, the optical element 204 only guides the beam 201 reflected from the polygon scanning mirror 202 (i.e. the beam 201 reflected at the varying angle α) towards the eye E of the subject during the rotation of the polygon scanning mirror 202 corresponding to the part β of the varying angle α for each facet. That is, as the polygon scanning mirror 202 reflects the beam 201 at the varying angle α, the beam 201 is only reflected by the optical element 204 towards the eye E of the subject when the polygon scanning mirror 202 reflects the beam 201 at the varying angle β due to the angle subtended by the arc of the optical element 204. Path "C" is an example of the beam 201 reflected from the active facet when first activated on the optical element 204 as the polygon scanning mirror 202 rotates. Path "D" is an example of the beam 201 reflected from the active facet 2021 when last incident on the optical element 204 as the polygon scanning mirror 202 rotates. Outside of this varying angle β, the beam L_{B} is thus not incident on the optical element 204 so does not reach the eye E. Therefore, no light, or a negligible amount thereof, may return from the eye E towards the polygon scanning mirror 202 to be detected by the detector 205.

As described above, the reflection process is repeated continuously as the polygon scanning mirror 202 operates and rotates and so different individual facets form each successive scan line. The ophthalmic imaging instrument 102 thus further comprises an aperture (i.e. an opening) 206 arranged to constrain (or limit) the returning light L_{R} reaching the detector 205 to be (only) that reflected from a same-size (and same-shape) region of each facet during rotation of the polygon scanning mirror corresponding to the (e.g. same) part β of the varying angle α for each facet. In other words, the aperture 206 is arranged to block some of the returning light L_{R} from the eye E such that the returning light L_{R} reflected from each facet is independent of a size of the respective facet during rotation of the polygon scanning mirror 202 corresponding to the part β of the varying angle α for each facet. Accordingly, as the reflection process is repeated, each facet reflects the beam 201 at the varying angle α and the aperture 206 constrains the returning light L_{R} reaching the detector 205 to be that reflected from the same-size region of the facet as the facet reflects the beam 201 at the varying angle β. The polygon scanning mirror 202 thereby includes a plurality of angular rotations within each revolution which each correspond to the polygon scanning mirror 202 reflecting the beam 201 through the varying angle β on a facet of the plurality of facets. As would be appreciated by those skilled in the art, the symmetric arrangement of the polygon scanning mirror 202 and the aperture 206 in Figure 2A cause the part β of the varying angle α for each facet to be the same, although the above-described respective part β of the varying angle α for each facet may differ depending on the size and shape of the facets and their relative orientations to the aperture 206 and beam 201. The same-size region extends the same first distance on each facet in its respective first direction and the same second distance on each facet in the common second direction.

The use of the aperture 206 thereby ensures that, during rotation of the polygon scanning mirror 202 corresponding to the part β of the varying angle α for each facet, the amount of light detected by the detector 205 will be the same independent of which facet is currently reflecting the beam 201 (and the returning light L_{R}). Variances in the size of each facet which may result in differing amounts of light being detected by the detector 205 to form an image of the eye E, as later described, are thereby eliminated by use of the aperture 206 during rotation of the polygon scanning mirror 202 corresponding to the part β of the varying angle α for each facet. Images formed from scan lines corresponding to the returning light L_{R} detected during the rotation of the polygon scanning mirror 202 corresponding to the part β of the varying angle α for each facet therefore may exhibit no, or greatly reduced, visual 'banding' and so the quality of the images may be greatly improved.

Figure 3A shows an enlarged schematic view of the rotating polygon mirror 202 along with an aperture 306 which may, as in the present device embodiment, be the aperture 206. These components are shown alongside additional optional elements which may form a part of the ophthalmic imaging instrument 102, as set out below. The aperture 306 is arranged so that the beam 201 and the returning light L_{R} pass through it to reflect from each facet of the polygon scanning mirror 202. The aperture 306 is further arranged to project the returning light L_{R} onto the same-size region of each facet so as to be reflected from the same-size region of each facet during the rotation of the polygon scanning mirror 202 corresponding to the part β of the varying angle α for each facet. The aperture 306 subsequently blocks some of the returning light L_{R} reflected from the facet so that only the returning light L_{R} reflected from the same-size region of the facet reaches the detector 205, as later described.

As shown in Figure 3B, which shows a schematic view of the rotating polygon mirror 202 and the aperture 306 in the x-y plane when the (active) facet 2021 is arranged parallel to the aperture 306, the aperture 306 is a rectangle in a plane 308 (which is parallel to the x-y plane). The aperture 306 has a length defined by the lengths of two edges 306-3, 306-4 parallel to the plane of rotation and a width defined by the lengths of two edges 306-1, 306-2 orthogonal to the plane of rotation. The line orthogonal to the plane 308 of the aperture 306 and passing the centre of the aperture 306 crosses the axis of rotation a_{X} of the polygon scanning mirror 202 and is parallel to the plane of rotation of the polygon scanning mirror 202. Further, each facet when reflecting the beam 201 is parallel to the plane 308 of the aperture 306 at a given rotation of the polygon scanning mirror 202. However, it would be appreciated by those skilled in the art that other geometries and arrangements of the aperture 306 may alternatively be used. The aperture 306 may, as in the present embodiment, be in a plate 307 (i.e. a hole in the plate 307), although it may alternatively be formed in a sheet or the like. The components forming the aperture 306 may be mounted within the ophthalmic imaging instrument 102 (e.g. to the housing of the ophthalmic imaging instrument 102). In some embodiments, the plate 307 may block the beam 201 reflected at the varying angle α for a portion of the varying angle α as the facet 2021 is moved into the path of the beam 201 from the light source 200. In such cases, the part β of the varying angle α may not include said portion of the varying angle α as no light, or a negligible amount thereof, may return from the eye E towards the polygon scanning mirror 202 to be detected by the detector 205 (as the beam 201 is not incident on the eye E).

As further shown in Figure 3B, the returning light L_{R} reaching the detector 205 is that projected onto and reflected from a central region 2021(A) on the facet 2021, which is the same-sized (and same-shaped) region of each facet. This central region 2021(A) extends in the same first distance 224 in the respective first direction d₁ (which in this case is the y-direction) on the facet 2021, and the same second distance 225 in the common second direction d₂ (which is the x-direction) on the facet 2021. The central region 2021(A) is surrounded by an outer peripheral region 2021(B) (shown as dashed in Figure 3B). The shape of the central region 2021(A) is determined by the shape of aperture 206 and, in this case, is rectangular. The central region 2021(A) is smaller than the size of the aperture 306 due to the angle of the beam 201 and the angle of the returning light L_{R}. That is, in this orientation of the facet 2021, the returning light L_{R} is blocked by the upper face of the plate 307 forming the aperture 306 from reflecting from the facet 2021 below the central region 2021(A) and the returning light L_{R} reflecting from the facet 2021 above the central region 2021(A) is blocked from reaching the detector 205 by the lower face of the plate 307.

During the rotation of the polygon scanning mirror 202 corresponding to the part β of the varying angle α for the facet 2021, the central region 2021(A) on the facet 2021 moves between a first bound 220 and a second bound 221 on the facet 2021. While the central region 2021(A) is within these bounds, it is the same size (and shape) independent of which facet the region is formed on for the equivalent angular rotation of the polygon scanning mirror 202 (i.e. as corresponding to an equivalent angle within the varying angle β for each facet). These bounds 220, 221 are a first predetermined distance 223 from the edges 2021-1, 2021-2 of the facet 2021 defining its width, although alternatively the bounds 220, 221 may be different distances from their respective nearest of these edges 2021-1, 2021-2.

This movement of the central region 2021(A) repeats as the polygon scanning mirror 202 rotates so as to reflect the returning light L_{R} from the next facet 2022 (not shown). In this way, the central region of each facet may be greater than or equal to a predetermined distance from the edges of the facet which may, for example, be either 0.1%, 1% or 10% of the width, or length, of the facet(s). As shown in Figure 3B, for the facet 2021 the central region 2021(A) is greater than or equal to the first predetermined distance 223 from the edges 2021-1, 2021-2 of the facet 2021 defining its width and is greater than or equal to a second predetermined distance 222 from the edges 2021-3, 2021-4 of the facet defining its length during rotation of the polygon scanning mirror 202 corresponding to the part β of the varying angle α for the facet 2021. Thus, the central region 2021(A) remains a distance from all edges 2021-1, ..., 2021-4 of the facet 2021 which is greater than or equal to the lesser of the first predetermined distance 223 and the second predetermined distance 222 (or both, in the case that the first predetermined distance 223 and the second predetermined distance 222 are the same). More generally (e.g. for other facet shapes), the separation (or gap) on each facet between the central region 2021(A) of the facet and the perimeter of the facet is greater than or equal to the predetermined distance. In this case, the predetermined distance may be either 0.1%, 1% or 10% of the distance extended by the facet in at least one of its respective first direction and/or the common second direction.

By ensuring that the central region of each facet is greater than or equal to the predetermined distance from the perimeter of the facet during rotation of the polygon scanning mirror 202 corresponding to the part β of the varying angle α for the facet, the aperture 206 constrains the returning light L_{R} reaching the detector 205 to be that reflected from a same-size region on each facet as this region on each facet is removed from the edges of the facet whereby the differences in size (e.g. length or width) of the facet would result in differences in the size of the region reflecting the returning light L_{R}. This ensures that the amount of light collected by the detector 205 will be the same regardless of which facet is the active facet, as set out above. Further, the predetermined distance from the edges of the facet may be set to avoid regions in the outer periphery of each facet which may contain variations in, for example, reflectivity of the facet as a result of the manufacturing process used to produce the polygon scanning mirror 202, which may further improve image quality.

Returning to Figure 3A, the ophthalmic imaging instrument 102 further comprises an optional housing 208 with an opening 209, the housing 208 enclosing the rotating polygon mirror 202. The housing 208 may protect the ophthalmic imaging instrument 102 from failure of the fast-spinning polygon scanning mirror 202 by containing pieces of the polygon scanning mirror 202 which may be propelled outwards at high velocities in such instances. Where the optional housing 208 is provided, the plate 307 forming the aperture 306 is attached to the housing 208 over the opening 209 so as to align (e.g. the centres of) the aperture 306 and opening 209. In this way, the beam of light L_{B} and the returning light L_{R} may pass through both the aperture 306 and the opening 209 to reflect from each facet of the polygon scanning mirror 202. However, where the housing 208 is omitted, the plate 307 may be otherwise attached to the ophthalmic imaging instrument 102 such as, for example, to the aforementioned housing of the ophthalmic imaging instrument 102. The opening 209 may, as in the present device embodiment, be larger than the size of each facet of the plurality of facets so as to not, or negligibly, constrain (or limit) the returning light L_{R} reaching the detector that is reflected from each (active) facet. However, in some embodiments, the aperture 206 may be provided by way of the opening 209 and so may be implemented directly by the housing 208 so as to reduce the ophthalmic imaging instrument's component count.

The ophthalmic imaging instrument 102 (or, more specifically, the opening 209) further comprises an optional phase mask 210 which may be arranged to cover the opening 209. The phase mask 210 may correct for aberrations caused by at least one of the optical element 204 and the later described components and optical elements 290, ... (e.g. scanner surfaces and ellipsoidal mirrors, as later described).

Whilst the aperture 306 is described above to be arranged so that the beam of light L_{B} and the returning light L_{R} pass through it to reflect from each facet of the polygon scanning mirror 202, and to project the returning light L_{R} onto the same-size region of each facet so as to be reflected from the same-size region of each facet during the rotation of the polygon scanning mirror 202 corresponding to the part β of the varying angle α for each facet, the present invention is not so limited. For example, in some embodiments the aperture 206 (or the aperture 306 thereof) may alternatively be placed within the path of the beam 201 between the polygon scanning mirror 202 and the light source 200 so as to constrain the returning light L_{R} reaching the detector 205 to be that reflected from a same-size region of each facet during the rotation of the polygon scanning mirror 202 corresponding to the part β of the varying angle α for each facet. In other words, the aperture 206 blocks the returning light L_{R} which reflects from outside of the same-size region of each facet from reaching the detector 205 without first projecting the returning light L_{R} onto from the same-size region of each facet (the returning light L_{R} thus being incident on the whole of each facet in this case). The aperture 206 in this case may, by way of example, be a circular aperture which is coaxial with the beam 201.

Further, whilst the aperture 206 (and the example aperture 306 thereof) is described above to constrain the returning light L_{R} reaching the detector 205 to be that reflected from a same-size (and same-shape) region of each facet, the present invention is not so limited. More generally, the aperture 206 may be arranged to constrain (or limit) the returning light L_{R} reaching the detector 205 to be (only) that reflected from a region of each facet which extends at least one of a same first distance on each facet in its respective first direction (of that facet) and/or a same second distance on each facet in the common second direction. That is, the region of each facet may extend the first distance in the respective first direction on that facet and this first distance is the same on all of the plurality of facets, and the region on each facet may extend the second distance in the common second direction on each facet and this second distance is the same on all of the plurality of facets. The aperture 206 may be similarly arranged, as described above, so that the beam 201 and the returning light L_{R} pass through it to reflect from each facet of the polygon scanning mirror 202, and to project the returning light L_{R} onto the region of each facet so as to be reflected from the region of each facet (which may, in some cases, be during the rotation of the polygon scanning mirror 202 corresponding to the part β of the varying angle α for each facet).

Thereby, in addition to the above-described example of the aperture 306, the aperture 206 may otherwise be, by way of example only: a slot in a plate (e.g. on the plane 308 in place of the aperture 306) extending in the y-direction so as to constrain the returning light L_{R} reaching the detector 205 in the common second direction on each facet along the full extent of each facet in the respective first direction (i.e. the facet's length) but to not constrain the returning light L_{R} reaching the detector 205 in the respective first direction on each facet; or a slot in a plate (e.g. on the plane 308 in place of the aperture 306) extending in the x-direction so as to constrain the returning light L_{R} reaching the detector in the respective first direction of each facet along the full extent of each facet in the second direction (i.e., the facet's width) but not constrain the returning light L_{R} reaching the detector 205 in the common second direction on each facet. In this way, the aperture 206 may constrain the returning light L_{R} reaching the detector 205 during rotation of the polygon scanning mirror 202 so as to reduce a variation among the plurality of facets 2021, 2022, ... in the amount of the returning light L_{R} reflected from each facet 2021, 2022, ... which reaches the detector 205. By reducing this variation, images formed from scan lines corresponding to the detected returning light L_{R} for each facet may exhibit reduced visual 'banding' and so the quality of the images may be improved.

By way of further example, where the aperture 206 is a slot in a plate extending in the y-direction, in Figure 3B the central region 2021(A) would instead extend in the respective first direction d₁ from the edge 2021-1 to the edge 2021-2 of the facet 2021. The aperture 206 may therefore constrain the returning light L_{R} reaching the detector 205 to be that reflected from a region extending the same second distance 225 on each facet in the common second direction d₂ which may thus reduce, or eliminate, the variations in the width of each facet which may occur from the manufacturing process so as to reduce, or remove, the visible 'banding' in the image, as previously described.

Returning to Figure 2A, the detector 205 is arranged to detect the returning light L_{R} reflected from the polygon scanning mirror 202 (i.e. from one or more of the facets) to generate a detection signal S_{d}. Thereby, the presence of the aperture 206 results in the detector 205 detecting the returning light L_{R} that is reflected from the same-sized region of each facet or, in the above-described more general case, the region of each facet. As described above, the detection signal S_{d} is transmitted to, and received by, the computing resources 106 via an access 207 to the computing resources 106. The detector 205 may comprise a one- or two-dimensional array of photo-sensing elements. However, the form of the detector 205 is not limited and the detector 205 may, for example, comprise a balanced photodetector arrangement comprising two reverse-biased photodiodes whose output photocurrents are subtracted from one another, the subtracted current signal being converted to a voltage detection signal by a transimpedance amplifier.

The access 207 is to the computing resources 106, the computing resources 106 being arranged to generate an image 210 of the eye E using the detection signal S_{d} generated from the returning light L_{R} reflected by at least one facet (and detected by the detector 205). The detection signal S_{d} used may, as in the present device embodiment, be that generated from the returning light L_{R} during rotation of the polygon scanning mirror 202 corresponding to the part β of the varying angle α for the at least one facet although, as later described, this is not necessarily the case. Thereby, the image 210 of the eye E is formed by the successive scan lines each corresponding to the detection signal S_{d} generated from the returning light L_{R} detected during rotation of the polygon scanning mirror 202 corresponding to the part β of the varying angle α for each facet. The amount of light detected by the detector 205 will thus be the same for each scan line regardless of which facet is currently reflecting the beam 201, as set out above, as in each scan line the returning light L_{R} is that reflected from the same-size region of the facet. In other words, the computing resources 106 are arranged to generate the image 210 of the eye E from the returning light L_{R} reflected from the same-size region of each facet. In some embodiments, the image 210 of the eye E is generated using the detection signal S_{D} generated from the returning light L_{R} reflected by all facets during rotation of the polygon scanning mirror 202 corresponding to the part β of the varying angle α for said facets. In other words, the image 210 comprises scan lines respectively corresponding to the reflection of the returning light L_{R} from each of the facets of the polygon scanning mirror 202 during the rotation of the polygon scanning mirror 202 corresponding to the part β of the varying angle α for each facet.

In the arrangement of Figure 2A, the detection signal S_{d} may be generated from the returning light L_{R} detected during the rotation of the polygon scanning mirror 202 corresponding to the whole varying angle α (thus including the part β). Accordingly, the access 207 to the computing resources 106 may ignore, or discard, the detection signal S_{d} generated from the returning light L_{R} reflected by the at least one facet during the rotation of the polygon scanning mirror 202 which does not correspond to the part β of the varying angle α for the at least one facet. Thus, the detection signal S_{d} used to generate the image 210 of the eye (E) is that generated during the rotation of the polygon scanning mirror 202 corresponding to a part β of the varying angle α for the at least one facet, and so the image 210 is generated using only the detection signal S_{d} generated from the returning light L_{R} reflected from the same-size region of the at least one facet or, in the above-described more general case, the region of the at least one facet.

In the alternative arrangement of Figure 2B, outside of the varying angle β the beam 201 does not reach the eye E and so no light returns to be detected by the detector 205, as previously described. Accordingly, there is no detection signal S_{d} generated during rotation of polygon scanning mirror 202 corresponding to the part of the varying angle α outside of the varying angle β. Thus, the only detection signal S_{d} used by the access 207 to the computing resources 106 to generate the image 210 is that generated from the returning light L_{R} reflected from the same-size region of the at least one facet or, in the above-described more general case, the region of the at least one facet.

In the case where the aperture 206 constrains the returning light L_{R} reaching the detector 205 to be that reflected from a region of each facet which extends the same second distance on each facet in the common second direction on that facet, the access 207 to the computing resources 106 may instead generate the image 210 of the eye E using the detection signal S_{d} generated from the returning light L_{R} reflected by at least one facet (and detected by the detector 205) during rotation of the polygon scanning mirror 202 corresponding to the varying angle α for the at least one facet. That is, in this case the part β of the varying angle α may be 100% of the varying angle α. In this way, each successive scan line in the image 210 may correspond to the detection signal S_{d} generated from the returning light L_{R} detected during rotation of the polygon scanning mirror 202 corresponding to the whole of varying angle α for each facet, allowing an extended field of view of the ophthalmic imaging instrument 120 to be obtained (via utilising the whole of each facet's length when reflecting the beam 201) whilst at least partially suppressing the visual 'banding' in the images.

Figure 4 shows a schematic view of an ophthalmic imaging instrument 120 especially in the context of examples of the further components and optical elements 290, ... according to an embodiment of the present invention and there are again shown elements as explained amongst others in conjunction with Figure 2 (same reference signs thus denote same elements and functionalities). The optional component shown in Figure 3A may be further included, as explained above. The ophthalmic imaging instrument 120 is described in the context of an imaging modality in which a beam of light 201 is generated by the light source 200 and guided toward a patient's eye E, reflected at tissue of the same and guided back to a detector 205. As part of an SLO module, the instrument 120 includes the light source 200 emitting a beam of light, that is, a scanning beam 201, and a plurality of scan relay elements. The scan relay elements include a first scanning element in the form of a polygon scanning mirror 202, a second scanning element 206, and optical elements that are positioned and configured to direct the scanning beam 201. In some embodiments, the optical elements may include the optical element 204 which may be a scan compensation element such as a curved mirror or slit mirror, and a second optical element 208 which may be a scan transfer element such as a main mirror.

The optical elements 204 and 208 are positioned and configured to direct the scanning beam 201. The second scanning element 206 may be or include an oscillating plane scanning mirror or a planar scanning mirror coupled to a galvanometer motor. The optical element 204 may be a curved mirror such as an ellipsoidal mirror. The second optical element 208 may be an aspherical mirror. It is to be appreciated that the first and second optical elements may have an alternative form. The scanning elements 202 and 206 may be referred to as a scanning device or a plurality of individual scanning devices. It is to be understood that the shown functionalities are just an example of a configuration that can be used with the embodiments described herein. In some examples, one or more of the scanning elements may include one or more of: an oscillating plane mirror, a galvanometer mirror, a MEMS mirror, a rotating mirror, prism or polygon scanner, and/or a resonant mirror, for example.

The aperture 206 is provided which is arranged to constrain the returning light L_{R} to be that reflected from a same-size region of each facet of the rotating polygon mirror 202 during the rotation of the polygon scanning mirror 202 corresponding to the part β of the varying angle α for each facet, as explained above.

In some examples, the detector 205 may be a separate component or device that is operatively coupled with the light source 200 such that the detector 205 and the light source 200 may be positioned at the same location or adjacent to one another. In some examples, the light source 200 may be implemented with the detector 205 as a single device, or devices within a common housing, such that in addition to emitting the scanning beam 201, the device may also be capable of detecting or receiving light, such as the light reflected back from the first scanning element 202 or from the optical element 204. In some further examples, the ophthalmic imaging instrument 120 may include a focussing lens placed before the detector 205 which is arranged to focus all, or substantially all, of the returning light L_{R} constrained by the aperture 206 onto the detector 205.

In an exemplary SLO imaging modality, the scanning beam 201 may be a laser of suitable wavelength as used in SLO applications. If other imaging modalities are included, the scanning beam 201 may be a collimated light which includes the laser for SLO applications and a superluminescent diode (SLD) for other applications, for example. It should be appreciated that any suitable source of collimated light may be used, such as a single frequency laser diode, vertical-cavity surface-emitting laser, wavelength swept laser source, pulsed laser source, or other source that has enough intensity and to be well collimated and produce adequate retinal illumination. In such applications, the SLD may be used due to the short coherence lengths required to discriminate the retinal layers from the resultant interferometric data. The SLD may be free space or fibre coupled into standard or polarization maintaining fibre to the scan system. A swept source laser may also be used in other applications, whereby the wavelength of the source is tuned over a given range.

In some embodiments, each of the first scanning element 202 or the second scanning element 206 may be a single element or an arrangement of two or more elements as suitable to provide a scan at a respective focal point F1 or F2, as shown, at which the scanning element is disposed. The focal points F1 and F2 are the foci of the optical element 204, and the focal points F2 and F3 are the foci of the optical element 208. The first scanning element 202 is positioned at focal point F1, the second scanning element 206 is positioned at focal point F2, and the eye E (e.g. the pupil of the eye E) is positioned at focal point F3 (also referred to as a virtual scanning point). The resulting scan may be a 2D scan or scan pattern of the scanning beam 201 as the light sweeps through the virtual scanning point (e.g. through F3) onto the eye E (e.g. onto the fundus of the eye E).

In some embodiments, the first scanning element 202 provides either a vertical, horizontal, or patterned scan which is incident on the optical element 204, to a point on the second scanning element 206 via the optical element 204. The scans may be 1D or 2D light scans, for example. The axes of the first scanning element 202 and the second scanning element 206 may be arranged to create a 2D light scan, such as in the form of a raster scan pattern of the scanning beam 201. The alignment of the first and second scanning elements 202, 206 may be orthogonal, substantially orthogonal, or arranged to generate an arbitrary scan geometry about the optical elements 204 and 208.

In some embodiments, the second scanning element 206 provides a plurality of scans, for example 1D or 2D light scans, which may comprise horizontal scans, vertical scans, or arbitrary patterns of the scanning beam 201. The scans provided by the first scanning element 202 and the scans provided by the second scanning element 206 are different from each other, for example with respect to the orientation of the scans. In some examples, one of the scanning elements may provide vertical scans of the retina, and the other scanning element may provide horizontal scans of the retina. The scanning beam 201 is directed towards the eye E via the scanning elements 202 and 206, and the optical elements 204 and 208, such that, for example, a wide-field, or an ultra-wide field, scan angle is achieved at the pupil plane of the eye E.

A "wide field" scanning refers to a scan angle in excess of 50 degrees in one or two dimensions. An "ultra-wide field" scanning refers to a scan covering substantially the entire retina of the eye E. In some examples, the plurality of line scans may be generated by scanning the retina along a first direction using the first scanning element 202, and positions of the plurality of line scans are varied along a second direction using the second scanning element 206, where the second direction is orthogonal to the first direction. As illustrated in Figure 4, the path of the scanning beam 201 is shown in a 1D scan produced by one oscillation or rotation (shown by the curved arrow) of the first scanning element 202. Path "A" is an example of the scanning beam 201 reflected from the polygon scanning mirror at one orientation of a reflecting facet during rotation, whereas paths "B" and "C" are examples of the scanning beam 201 reflected at other orientations of the facet during rotation.

The components of the ophthalmic imaging instrument 120 may be arranged such that the rotational axis of the first scanning element 202 is substantially parallel to a line joining the two foci of the optical element 208 (i.e., F2 and F3) such that the scanning beam 201 is scanned across the secondary axis of the optical element 204. Furthermore, the first scanning element 202 may produce a 1D or 2D scan which is incident on the optical element 204. The optical element 204 may also therefore produce a 1D or 2D scan. The components of the ophthalmic imaging instrument 102 may be arranged such that the line joining the two foci of the optical element 208 (i.e., F2 and F3) lies substantially on a plane defined by the scan (e.g., 1D vertical scan) produced by the optical element 204.

The first and second scanning elements 202 and 206 may thus together create a light scan, for example a 2D scan, in the form of a raster scan pattern from a single point in space at or near the focal point F3 at or in the eye E of the patient. The first and second scanning elements may have operating parameters which include the amplitude of the oscillation and the rotational offset of the oscillation. The operating parameters also include the velocity of oscillation. Both of these operating parameters may be selected to control the direction and pattern of the light scan from the apparent point source. In some examples, the first and second scanning elements may be housed in a rotation mount (not shown) that can adjust the centring (or eccentricity) of the scanning beam 201 on the retina of the eye E, which provides the ability to "move" the imaging field across the retina.

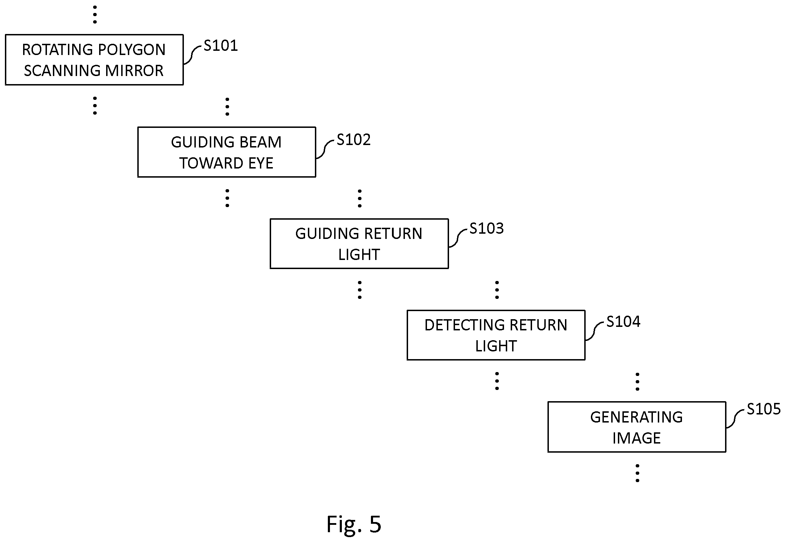

Figure 5 shows a flowchart of a general method embodiment of the present invention. Specifically, the method embodiments relate to operating an ophthalmic imaging instrument that comprises a light source, a polygon scanning mirror, an optical element, a detector, an aperture and an access to computing resources. For example, the methods may be suitable for operating the ophthalmic imaging instrument 102 as disclosed and described in conjunctions with embodiments of the present disclosure. The method comprises a step S101 of rotating, by means of the driver, said polygon scanning mirror such that each facet reflects a beam of light (at a varying angle), a step S102 of guiding, by means of the optical element, the beam reflected (at the varying angle) from the polygon scanning mirror toward an eye of a subject, a step S103 of guiding, by means of the optical element, light returning from the eye towards the polygon scanning mirror, a step S104 of generating, by means of the detector, the detection signal by detecting the returning light constrained by the aperture and a step S105 of generating, by means of the access to the computing resources, an image of the eye using the detection signal generated from the returning light reflected from at least one facet (during rotation of the polygon scanning mirror corresponding to the part of the varying angle for the at least one facet). It is noted that the steps S101 to S105 can be performed concurrently and/or at least in part sequentially as is most suitable for the chosen implementation.

The forgoing has presented embodiments and details thereof as part of the present invention which can provide one or more advantages by improving power control mechanisms in ophthalmic imaging instruments that allow for the flexibility of providing a plurality of imaging modalities but maintain safety while not requiring an increase in system and/or implementation. While various embodiments of the present disclosure have been described above, it should be understood that they have been presented by way of example, not limitation. It will be apparent to persons skilled in the relevant art(s) that various changes in form and detail can be made therein. Thus, the above-described exemplary embodiments are not limiting.

While this specification contains many specific embodiment details, these should not be construed as limitations on the scope of any inventions or of what may be claimed, but rather as descriptions of features specific to particular embodiments described herein. Certain features that are described in this specification in the context of separate embodiments can also be implemented in combination in a single embodiment. Conversely, various features that are described in the context of a single embodiment can also be implemented in multiple embodiments separately or in any suitable sub-combination. Moreover, although features may be described above as acting in certain combinations and even initially claimed as such, one or more features from a claimed combination can in some cases be excised from the combination, and the claimed combination may be directed to a sub-combination or variation of a sub-combination.

## Claims

1. An ophthalmic imaging instrument (102, 120) comprising:
a light source (200) arranged to emit a beam of light (201);
a polygon scanning mirror (202) comprising a plurality of reflecting facets (2021, 2022, ...) each extending in a respective first direction (d₁) in a plane of rotation of the polygon scanning mirror (202) and in a common second direction (d₂) orthogonal to the plane of rotation of the polygon scanning mirror (202);
a driver (203) arranged to rotate during operation said polygon scanning mirror (202) in the plane of rotation, such that each facet (2021, 2022, ...) reflects the beam of light (201);
an optical element (204) arranged to guide the beam (201) reflected from the polygon scanning mirror (201) towards an eye (E) of a subject and to guide light (L_{R}) returning from the eye (E) towards the polygon scanning mirror (202);
a detector (205) arranged to detect the returning light (L_{R}) reflected from the polygon scanning mirror (202) to generate a detection signal (S_{d});
an aperture (206) arranged to constrain the returning light (L_{R}) reaching the detector (205) to be that reflected from a region (2021(A)) of each facet (2021, 2022, ...) which extends at least one of a same first distance on each facet in its respective first direction (d₁) and a same second distance on each facet in the common second direction (d₂); and
an access (207) to computing resources (106) which are arranged to generate an image (210) of the eye (E) using the detection signal (S_{d}) generated from the returning light (L_{R}) reflected by at least one facet (2021, 2022, ...).

2. The ophthalmic imaging apparatus of Claim 1, wherein the region (2021(A)) of each facet (2021, 2022, ...) is a same-sized region which extends the same first distance on each facet in its respective first direction (d₁) and the same second distance on each facet in the common second direction (d₂).

3. The ophthalmic imaging instrument (102, 120) of Claim 2, wherein the driver (203) rotates during operation the polygon scanning mirror (202) in the plane of rotation, such that each facet (2021, 2022, ...) reflects the beam of light (201) at a varying angle (α), and wherein the optical element (204) only guides the beam (201) reflected from the polygon scanning mirror (202) towards the eye (E) of the subject during the rotation of the polygon scanning mirror (202) corresponding to a same part (β) of the varying angle (α) for each facet (2021, 2022, ...).

4. The ophthalmic imaging instrument (102, 120) of any of Claims 1 to 2, wherein the driver (203) rotates during operation the polygon scanning mirror (202) in the plane of rotation, such that each facet (2021, 2022, ...) reflects the beam of light (201) at a varying angle (α), and wherein the detection signal (S_{d}) used to generate the image (210) of the eye (E) is that generated during the rotation of the polygon scanning mirror (202) corresponding to a respective part (β) of the varying angle (α) for the at least one facet (2021, 2022, ...), and the detection signal (S_{d}) not used to generate the image (210) of the eye (E) is ignored by the computing resources (106).

5. The ophthalmic imaging instrument (102, 120) of any preceding claim, wherein the aperture (206) is further arranged so that the beam of light (201) and the returning light (L_{R}) pass through it to reflect from each facet (2021, 2022, ...) of the polygon scanning mirror (202), and to project the returning light (L_{R}) onto the region (2021(A)) of each facet so as to be reflected from the region (2021(A)) of each facet.

6. The ophthalmic imaging instrument (102, 120) of any preceding claim, wherein the plurality of facets (2021, 2022, ...) are rectangular, each facet has a length defined by lengths of two edges (2021-3, 2021-4) which are parallel to the plane of rotation and a width defined by lengths of two edges (2021-1, 2021-2) which are orthogonal to the plane of rotation, and the aperture (206) is rectangular, having a length defined by lengths of two edges (206-3, 206-4) which are parallel to the plane of rotation and a width defined by lengths of two edges (206-1, 206-2) which are orthogonal to the plane of rotation.

7. The ophthalmic imaging instrument (102, 120) of any preceding claim, wherein the region (2021(A)) of each facet (2021, 2022, ...) is a central region (2021(A)) on the facet surrounded by an outer peripheral region (2021(B)) on the facet.

8. The ophthalmic imaging instrument (102, 120) of Claim 7, wherein the separation on each facet between the central region (2021(A)) of the facet and the perimeter of the facet is greater than or equal to a predetermined distance (222, 223).

9. The ophthalmic imaging instrument (102, 120) of Claim 8, wherein the predetermined distance (222, 223) is either 0.1%, 1% or 10% of the distance extended by the facet in at least one of its respective first direction (d₁) and the common second direction (d₂).

10. The ophthalmic imaging instrument (102, 120) of any preceding claim, wherein the polygon scanning mirror (202) is enclosed by a housing (208) with an opening (209).

11. The ophthalmic imaging instrument (102, 120) of Claim 10, wherein the opening (209) comprises a phase mask (210).

12. The ophthalmic imaging instrument (102, 120) of Claim 10 or Claim 11, wherein the aperture (206) is in a plate (207) and the plate (207) is attached to the housing (208) over the opening (209) of the housing (208) so as to align the aperture (206) and the opening (209).

13. The ophthalmic imaging instrument (102, 120) of any preceding claim, wherein the image (210) of the eye (E) is generated using the detection signal (S_{d}) generated from the returning light (L_{R}) reflected by all facets (2021, 2022, ...) during rotation of the polygon scanning mirror (202).

14. The ophthalmic imaging instrument (102, 120) of any preceding claim, wherein the polygon scanning mirror (202) is a regular convex polygon with 16 facets.

15. A method for operating an ophthalmic imaging instrument (102, 120) according to any preceding claim, the method comprising:
- rotating (S101), by means of the driver (203), said polygon scanning mirror (202) such that each facet (2021, 2022, ...) reflects the beam of light (201);
- guiding (S102), by means of the optical element (204), the beam (201) reflected from the polygon scanning mirror (202) towards the eye (E) of the subject;
- guiding (S103), by means of the optical element (204), light (L_{R}) returning from the eye (E) towards the polygon scanning mirror (202);
- generating (S104), by means of the detector (205), the detection signal (S_{d}) by detecting the returning light (L_{R}) constrained by the aperture (206); and
- generating (S105), by means of the computing resources (106), the image (210) of the eye (E) using the detection signal (S_{d}) generated from the returning light (L_{R}) reflected from at least one facet.
